# EUROPEAN PATENT APPLICATION

(11) **EP 3 323 375 A1**
(43) Date of publication of application: **23.05.2018**
(21) Application number: 17202696.5
(22) Date of filing: 21.11.2017
(51) Int. Cl.: A61B 90/98

(54) **AN RFID TAG MOUNTED OR MOUNTABLE ON SURGICAL OR MEDICAL EQUIPMENT OR ON A SURGICAL OR MEDICAL INSTRUMENT**

(30) Priority: 22.11.2016 NL 2017833
(71) Applicant: Van Straten Medical B.V., 3439 MN Nieuwegein (NL)
(72) Inventor: VAN STRATEN, Bart Jan, 3439 MN Nieuwegein (NL); VAN STRATEN, Niels, 3439 MN Nieuwegein (NL); VAN STRATEN, Jaap, 3439 MN Nieuwegein (NL)
(74) Representative: Van Breda, Jacobus

(57) **Abstract**

An RFID tag (1) mounted or mountable on surgical or medical equipment or on a surgical or medical instrument (2) comprising:
- a metal ring shaped holder (3),
- a housing (4) made of an electrically nonconductive or slightly conductive material, and
- an RFID element (5) embedded in the housing (4),
- wherein the housing (4) is substantially or essentially contained within the metal ring shaped holder (3), and wherein
- the metal ring shaped holder is mounted on a support that is removably connectable to the surgical or medical equipment or instrument.

## Description

The invention relates to an RFID tag mounted or mountable on surgical or medical equipment or on a surgical or medical instrument comprising:
- a metal ring shaped holder,
- a housing for the RFID element made of an electrically nonconductive or slightly conductive material, and
- an RFID element embedded in the housing;
- wherein the housing is substantially or essentially contained within the metal ring shaped holder.

US 9,033,251 teaches an RFID tag comprising a metal ring shaped holder, a housing made of an electrically nonconductive or slightly conductive material, and an RFID element embedded in the housing. US 9,033,251 mentions as advantages that surgical instruments on which RFID tags are mounted are easy identifiable and traceable, without requiring any particular skill of the personnel. A notable advantage is that instruments with RFID tags mounted thereon reduce the risk that instruments remain in the body of the patient after surgery. Furthermore inventory and maintenance management can be improved when RFID tags are applied on the surgical or medical instruments and equipment. Examples are medical or rigid endoscopes and surgical power tools, but this is not an exhaustive enumeration.

WO2014/045265 teaches another RFID tag for a medical application. The RFID tag comprises a metal base to be suitably attached to a medical object to be marked, a receiving element secured to the metal base for holding the RFID element, and means for holding the RFID element at a determined distance from the base.

US2006/0214791 teaches an RFID tag that is integrated into a button type substantially cylindrically shaped housing with a portion for attaching the tag to an object to be identified. The portion for attaching the tag is provided with screw thread.

A further RFID tag for marking an object is taught by FR-A- 2 918 769. The RFID tag is directly provided on the object to be marked.

A disadvantage of all known RFID tags is that they at least intrinsically involve a modifying processing of the object to be marked, however slight this may be. For instance when the metal ring shaped holder of the known RFID tag is mounted on the object this requires either gluing or welding of the medical or surgical equipment or instrument.

It is an object of the invention to provide an RFID tag which can be applied without a modifying processing of the medical or surgical equipment or instrument to be marked, wherein the RFID tag can be easily removed or replaced.

The RFID tag of the invention has the features of one or more of the appended claims.

In a first aspect of the invention the RFID tag which comprises:
- a metal ring shaped holder,
- a housing made of an electrically nonconductive or slightly conductive material, and
- an RFID element embedded in the housing, wherein the housing is substantially or essentially contained within the metal ring shaped holder, is provided with the feature that the metal ring shaped holder is fixed on a support to form a unitary device which is removably connectable to the surgical or medical equipment or instrument.

This provides the advantage that the RFID tag can be simply clamped or attached to the surgical or medical equipment or instrument, without the latter being required to undergo a modifying treatment such as gluing or welding. In fact the unitary device is removably connectable to the surgical or medical equipment or instrument without applying any auxiliary means at all. An important advantage thereof is also that removal or replacement of the RFID tag is no big effort anymore as opposed to the prior art solutions.

Preferably the support has two legs that can engage and clamp to opposite sides of the surgical or medical equipment or instrument.

In one preferred embodiment at a far end of the housing the two legs each have a thickened portion for clamping the surgical or medical equipment or instrument.

In another preferred embodiment the support has two curved legs on opposite sides of the housing that merge into each other at a far end of the housing to embody the support as a ring itself.

Although in the prior art the housing is said to be of plastic or ceramic, these are not the only possible materials. In certain embodiments it is preferred to apply epoxy resin for the housing. Epoxy resin is relatively cheap and is easy to mold into the required shape without leaving undesired air holes in the material or adjacent to the RFID element. Suitably PEEK or POM is used as suggested by WO2014/045265.

The invention is also embodied in surgical or medical equipment or a surgical or medical instrument provided with an RFID tag according to the invention. Non-limiting examples are medical or rigid endoscopes or surgical power tools.

The invention will hereinafter be further elucidated with reference to the drawing of exemplary embodiments of an application of a RFID tag according to the invention that is not limiting as to the appended claims.

In the drawing:
- figure 1 shows a first embodiment of an RFID tag according to the invention;
- figure 2 shows the RFID tag of figure 1 placed on a medical object;
- figure 3 shows a second embodiment of the RFID tag according to the invention; and
- figure 4 shows the RFID tag of figure 3 provided on a medical object.

Whenever in the figures the same reference numerals are applied, these numerals refer to the same parts.

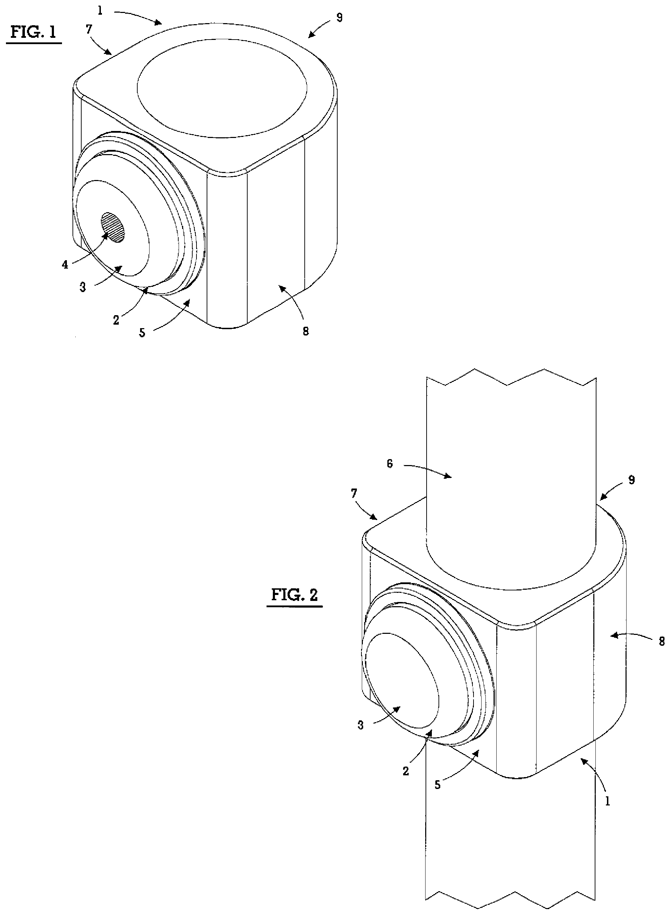

In the figures 1 - 4 the shown RFID tag 1 of the invention comprises:
- a metal ring shaped holder 2,
- a housing 3 made of an electrically nonconductive or slightly conductive material, and
- an RFID element 4 embedded in the housing 3. Another feature is that the housing 3 is substantially or essentially contained within the metal ring shaped holder 2. The housing 3 can be of several materials, such as plastic or ceramic, however it is preferred that the housing 4 is of epoxy resin, in particular PEEK or POM.

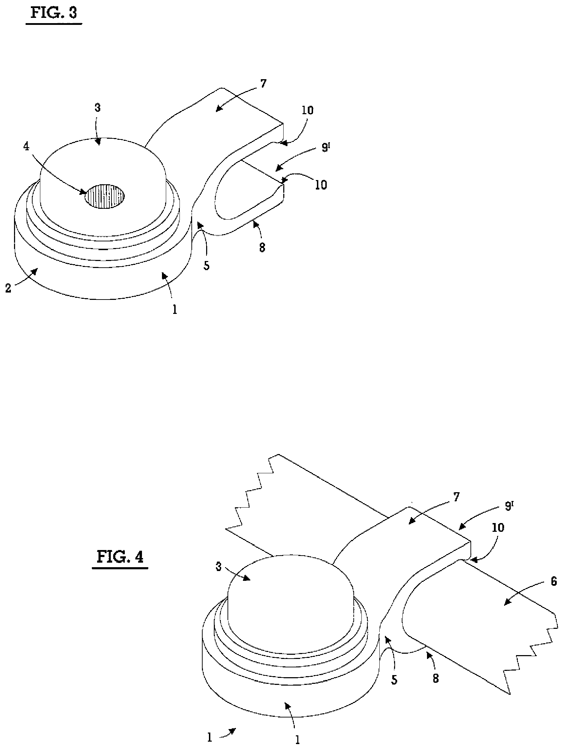

The metal ring shaped holder 2 is mounted on a support 5 to form a unitary device that is removably connectable to the surgical or medical equipment or instrument to be marked. This is for instance shown in figure 2 and in figure 4 showing a part 6 of some surgical or medical equipment or instrument on which the RFID tag 1 of the invention is applied without any auxiliary means being required for mounting of the RFID tag 1, which is embodied as a unitary device.

The inventors envisage several possible embodiments of the RFID tag of the invention, which share the feature that the support 5 has two legs 7, 8 that can engage and clamp to opposite sides of the surgical or medical equipment or instrument.

Figures 1 and 2 show an embodiment in which the support 5 has two curved legs 7, 8 on opposite sides of the metal ring 2 and the housing 3 contained therein, which two legs 7, 8 merge into each other at a far end 9 of the housing 3.

Figures 3 and 4 show another embodiment in which at a far end 9' of the housing 3 the two legs 7, 8 each have a thickened portion 10 that provides a click on arrangement for clamping the surgical or medical equipment or instrument to be marked by the RFID tag.

Although the invention has been discussed in the foregoing with reference to exemplary embodiments of the RFID tag of the invention and its use, the invention is not restricted to these particular embodiments which can be varied in many ways without departing from the invention. The discussed exemplary embodiments shall therefore not be used to construe the appended claims strictly in accordance therewith. On the contrary the embodiments are merely intended to explain the wording of the appended claims without intent to limit the claims. The scope of protection of the invention shall therefore be construed in accordance with the appended claims only, wherein a possible ambiguity in the wording of the claims shall be resolved using these exemplary embodiments.

## Claims

1. An RFID tag (1) mounted or mountable on surgical or medical equipment or on a surgical or medical instrument (6) comprising:
- a metal ring shaped holder (2),
- a housing (3) made of an electrically nonconductive or slightly conductive material, and
- an RFID element (4) embedded in the housing (3),
- wherein the housing (3) is substantially or essentially contained within the metal ring shaped holder (2), **characterized in that**
- the metal ring shaped holder (2) is fixed on a support (5) to form a unitary device which is removably connectable to the surgical or medical equipment or instrument (6).

2. The RFID tag of claim 1, **characterized in that** the unitary device is removably connectable to the surgical or medical equipment or instrument (6) without auxiliary means.

3. The RFID tag of claim 1 or 2, **characterized in that** the support (5) has two legs (7, 8) that can engage and clamp to opposite sides of the surgical or medical equipment or instrument (6).

4. The RFID tag of claim 3, **characterized in that** at a far end (9, 9') of the housing (3) the two legs (7, 8) each have a thickened portion (10) for clamping the surgical or medical equipment or instrument (6).

5. The RFID tag of claim 1, 2 or 3, **characterized in that** the support (5) has two curved legs (7, 8) on an opposite side of the housing (3) that merge into each other at a far end (9) of the housing (3).

6. The RFID tag of any one of claims 1 - 5, **characterized in that** the housing (3) is made of an epoxy resin, preferably PEEK or POM.

7. Surgical or medical equipment or instrument (2) provided with an RFID tag (1) according to any one of claims 1 - 6.
